# EUROPÄISCHE PATENTSCHRIFT

(11) **EP 0 435 825 B1**
(45) Veröffentlichungstag und Bekanntmachung des Hinweises auf die Patenterteilung: **17.01.1996**
(21) Anmeldenummer: 90811001.8
(22) Anmeldetag: 18.12.1990
(51) Int. Cl.: A61N 5/06

(54) **Vorrichtung zum Homogenisieren der inhomogenen Lichtverteilung eines Laserstrahllichtbündels**
Apparatus for homogenizing the inhomogeneous light distribution of a laser light beam
Appareil pour homogénéiser la distribution de lumière non-homogène d'un faisceau de lumière laser

(30) Priorität: 27.12.1989 CH 4652/89
(43) Veröffentlichungstag der Anmeldung: 03.07.1991
(73) Patentinhaber: CIBA-GEIGY AG, CH-4002 Basel (CH)
(72) Erfinder: Van den Bergh, Hubert, Dr., CH-1376 Goumoens-la-Ville (CH); Cornaz, Peter François, Prof. Dr., - (CH); Wagnières, Georges, CH-1095 Lutry (CH)

(56) Entgegenhaltungen:
- EP-A- 0 184 643
- WO-A-86/06817
- FR-A- 2 455 300
- US-A- 4 681 396
- Patent Abstract of Japan, Vol.8,No.144,(p-284)[1581], Infrared light waveguide

## Beschreibung

Die Erfindung betrifft eine Vorrichtung zum Homogenisieren der inhomogenen Lichtverteilung eines Laserstrahllichtbündels für eine scharfrandige Bestrahlung einer Fläche, mit einer vom Laserstrahllichtbündel beaufschlagten, eine Multimodefaser aufweisenden optischen Anordnung.

Es ist bekannt, das Licht eines Lasers mit Hilfe einer optischen Faser, z.B. einer Multimodefaser, zu sammeln und an einen vom Laser entfernt liegenden Ort zu bringen, um diesen mit dem von der Endfläche der Multimodefaser ausgehenden Licht zu beleuchten. Ein dafür geeignetes System, das zur Materialbearheitung eingesetzt wird, ist in der US-A-4,681,396 beschrieben. In diesem System wird das aus einem Laser austretende Laserstrahllichtbündel auf den Kern des eingangsseitigen Endes einer optischen Faser fokussiert und so in den Faserkern eingekoppelt. Nachdem das Licht die Faser durchlaufen hat, tritt es durch das ausgangsseitige Ende der Faser wieder aus und wird auf das zu bearbeitende Material fokussiert, die Abmessungen des Lichtflecks sind also sehr klein. Ausserdem weist das an der ausgangsseitigen Endfläche der Multimodefaser divergent austretende Laserstrahllichtbündel bei diesem System wie auch bei anderen üblichen Anordnungen eine inhomogene Intensitätsverteilung auf, wobei die Intensität entlang der Achse des Laserstrahllichtbündels maximal ist und nach den Seiten hin allmählich abfällt. Für die photodynamische Therapie von Hautkrebs sowie zur in vitro Bestrahlung von Zellkulturen ist es aber wünschenswert, eine gegenüber der Endfläche einer Multimodefaser grosse Fläche gleichmässig und präzise zu bestrahlen, um konstante und gut reproduzierbare Resultate zu erhalten. Ein scharfer Abfall der Lichtintensität seitlich des Lichtbündels ist insbesondere dann erforderlich, wenn die Selektivität der verwendeten chemischen Substanz klein ist, um eine Schädigung des gesunden Gewebes neben dem Tumor zu vermeiden. Ausserdem wird auf diese Weise Lichtenergie gespart.

Eine Vorrichtung gemäß Oberbegriff von Anspruch 1 ist auch aus FR-A-2455300 bekannt.

Der Erfindung liegt die Aufgabe zugrunde, eine Vorrichtung der eingangs genannten Art zu schaffen, die es gestattet, einen gegenüber der Querschnittsfläche einer Multimodefaser grosse Fläche mit einer optischen Dosis sehr gleichmässig verteilt und sehr präzise lokalisiert zu bestrahlen.

Diese Aufgabe wird erfindungsgemäss durch die Merkmale des kennzeichnenden Teils von Anspruch 1 gelöst.

Dadurch, dass die ausgangsseitige Endfläche nicht unmittelbar an die zu bestrahlende Fläche herangeführt ist, ergibt sich die Möglichkeit einer grobflächigeren Ausleuchtung, wobei die Projektionsoptik für eine randscharfe Ausleuchtung sorgt, indem sie die ausgangsseitige Endfläche auf die zu bestrahlende Fläche abbildet. Die ausgangsseitige Endfläche und die zu bestrahlende Fläche sind sehr homogen ausgeleuchtet weil in der einige Meter langen Multimodefaser eine Modenmischung erfolgt, so dass am Ende der Multimodefaser über deren Querschnitt verteilt eine homogene Energieverteilung vorliegt.

Die erfindungsgemässe Vorrichtung gestattet kurze Bestrahlungsabstände, was in manchen klinischen Situationen sehr hilfreich ist.

Bei einem zweckmässigen Ausführungsbeispiel der Erfindung erfolgt die Projektion mit Hilfe eine Mikroskopes, dessen Objektiv in geringem Abstand vor der Endfläche der Multimodefaser in der Weise angeordnet ist, dass die Langsachse der Multimodefaser mit der Längsachse des Mikroskopobjektives fluchtet. Bei dem Mikroskopobjektiv handelt es sich um ein Objektiv mit einer grösseren numerischen Apertur als diejenige der Multimodefaser, vorzugsweise um ein Objektiv mit einer numerischen Apertur von etwa 0,45, das es gestattet, von der ausgangsseitigen Endfläche der Multimodefaser ein zehn- bis hundertfach, insbesondere ein zwanzigfach vergrössertes Zwischenbild zu erzeugen. Das Zwischenbild wird bei einem Ausführungsbeispiel der Erfindung anschliessend mit Hilfe des Okulars des Mikroskopes mit einer zwölffachen Vergrösserung auf die zu bestrahlende Fläche abgebildet, so dass bei einem Kerndurchmesser von 200 Mikrometer für die Multimodefaser eine Scheibe von 4,8 cm Durchmesser mit einer rechteckförmigen Intensitätsverteilung ausgeleuchtet wird.

Als Fokussieroptik zum Einspeisen des Laserlichtes kann ein Mikroskopobjektiv mit einer numerischen Apertur von 0,45 und einem Vergrösserungsfaktor von 20 verwendet werden. Um ein scharfes Bild auf der auszuleuchtenden Fläche zu erhalten und um Abstandsänderungen auszugleichen, ist das Mikroskop mit Einrichtungen zum Grobjustieren und Feinjustieren versehen. Die Projektionsoptik ist dabei zusammen mit dem die ausgangsseitige Endfläche aufweisenden Abschnitt der Multimodefaser an einer Halteeinrichtung befestigt, die in axialer Richtung gegenüber der zu beleuchtenden Fläche verschiebbar ist, um den vorgegebenen Abstand für eine vorgegebene Grösse der ausgeleuchteten Fläche einzustellen.

Die Multimodefaser ist vorzugsweise in einem mittleren Abschnitt zu einer platzsparenden Spirale aufgewickelt. Eine besonders hohe Homogenität der Bestrahlungsdosis lässt sich erreichen, wenn in einem Abschnitt zwischen der Stirnfläche und der Endfläche der Multimodefaser eine Vibrationseinrichtung die Multimodefaser in Biegeschwingungen versetzt. Eine derartige Vibrationseinrichtung, wie aus Patent Abstract of Japan bekannt, Vol. 8, No. 144, (P-284) [1581] "Infrared light waveguide", kann aus einer rotierend hin- und herschwingenden Welle bestehen, deren Längsachse zu einem kurzen Abschnitt der Multimodefaser parallel verläuft und an ihrem freien Ende mit dem Mantel der Multimodefaser verbunden ist. Durch die Vibration und das Rütteln erfolgt durch eine zeitliche Mittelung eine Homogenisierung der Speckels und damit auch über relativ kürzere Bestrahlungszeiten eine hohe optische Dosishomogenität. Für das Auge eines Betrachters ergibt sich ein homogener speckelfreier Eindruck, wenn die Vibration eine das zeitliche Auflösungsvermögen des Auges übersteigende Frequenz hat.

Die Erfindung wird nachfolgend anhand der lediglich eine Figur umfassenden Zeichnung näher beschrieben, die in schematischer Darstellung eine Seitenansicht der Vorrichtung zum Homogenisieren der optischen Dosis bei grossflächiger Bestrahlung zeigt.

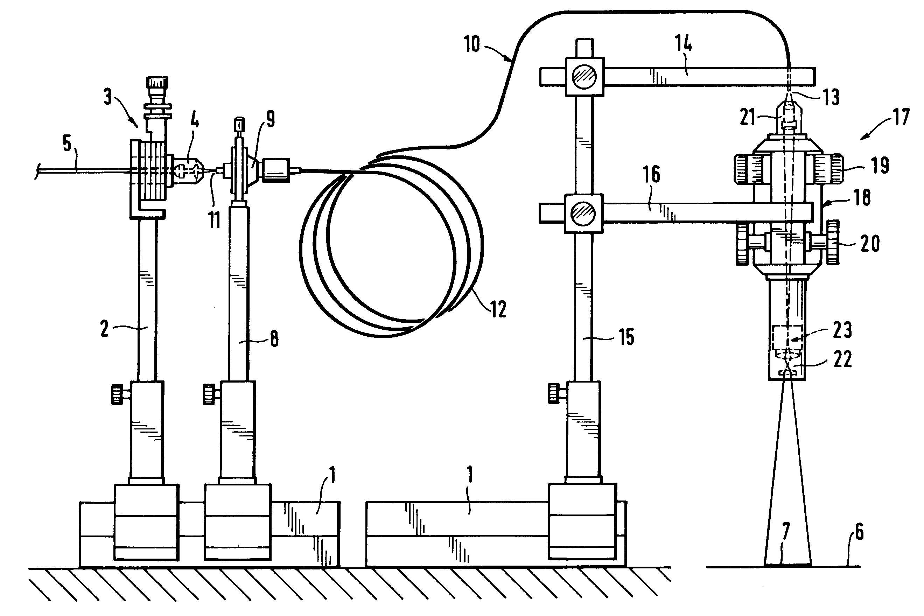

In der Zeichnung erkennt man Schienen einer optischen Bank 1, die zum Aufbau der Vorrichtung für eine grossflächige und homogene Bestrahlung eingesetzt werden kann. Ein erster Halter 2 auf der optischen Bank 1 trägt eine XY-Positioniereinrichtung 3 für den Halter eines Mikroskopobjektives 4, das beispielsweise eine numerische Apertur von 0,45 und eine Vergrösserung von 20 hat.

Das Mikroskopobjektiv 4 dient zum Fokussieren eines Laserstrahllichtbündels 5, dessen Intensitätsverteilung im Querschnitt im allgemeinen glockenförmig ist, so dass die Intensität in der Mitte des Laserstrahllichtbündels am höchsten ist und zum Rande hin allmählich abfällt. Die in der Zeichnung dargestellte Vorrichtung dient dazu, die glockenförmige Intensitätsverteilung über den Querschnitt des engen Laserstrahllichtbündels so umzusetzen, dass auf der Arbeitsfläche 6 eine bestrahlte Fläche 7 mit einem Durchmesser von mehreren Zentimetern homogen gleichmässig ausgeleuchtet ist und am Rand der bestrahlten Fläche ein sprunghafter Intensitätsabfall entsprechend einer rechteckförmigen Intensitätsverteilung vorliegt.

Ein zweiter Halter 8 auf der optischen Bank 1 verfügt über eine XY-Positioniereinrichtung mit einem Faserhalter 9 für das erste Ende einer Multimodefaser 10. Die Multimodefaser 10 hat beispielsweise einen Kerndurchmesser von 200 Mikrometern und einen Manteldurchmesser von 280 Mikrometern. Ihre numerische Apertur beträgt etwa 0,21. Eine besonders homogene Ausleuchtung der Fläche 7 ergibt sich, wenn eine Stufenindexfaser verwendet wird.

Die eingangsseitige Stirnfläche 11 des im Faserhalter 9 eingespannten ersten Endes der Multimodefaser 10 befindet sich in einem Abstand vom Mikroskopobjektiv 4, der geringfügig grösser ist als der Abstand der engsten Stelle des aus dem Mikroskopobjektiv 4 austretenden fokussierten Laserstrahllichtbündels, um eine optimale Lichteinspeisung und Justierung zu gestatten.

Die in die Stirnfläche 11 eingespeiste Lichtenergie pflanzt sich in der Multimodefaser 10 fort, die beispielsweise eine Länge von etwa 5 Metern aufweist und teilweise in Gestalt einer Spirale 12 ausgelegt sein kann. Infolge der groben Länge und der Krümmungen der Multimodefaser 10 erfolgt in deren Kern eine Modenvermischung, so dass an der ausgangsseitigen Endfläche 13 der Multimodefaser 10 eine gleichmässige Lichtintensitätsverteilung über die Querschnittsfläche des Kerns der Multimodefaser 10 beobachtet werden kann.

Eine besonders hohe mittlere Homogenität kann durch zeitliches Mitteln der Speckels erreicht werden, indem die Stufenindexfaser oder Multimodefaser 10 im Bereich der Spirale 12 oder an einer anderen Stelle mit Hilfe eines in der Zeichnung nicht dargestellten Vibrators in Schwingungen, insbesondere Biegungsschwingungen versetzt wird. Der Vibrator kann dabei beispielsweise in der Mitte der Multimodefaser 10 angeordnet sein und eine elektrisch betriebene hin und her rotierend schwingende Welle aufweisen, die mit ihrem parallel zur Multimodefaser 10 verlaufenden Ende mechanisch mit dem Mantel der Multimodefaser 10 gekoppelt ist. Als Einrichtung für eine derartige weitere Verbesserung der Homogenität der Lichtverteilung an der Endfläche 13 durch Elimination der Speckels kann der Antrieb einer elektrischen Zehnbürste verwendet werden, der batteriebetrieben und dadurch kompakt und unabhängig von der übrigen Anordnung ist.

Die Verbindung des Mantels der Multimodefaser 10 mit dem schwingend rotierenden Zapfen des Zahnbürstenantriebs kann dabei durch eine einige Zentimeter lange Leiste erfolgen, die an ihrem einen Ende auf den Zapfen der Zahnbürste aufgesteckt ist und auf der anderen Seite mit einer Klemme versehen ist, die den Mantel der Multimodefaser 10 schwingend mitnimmt.

Das zweite Ende der Multimodefaser 10 mit der Endfläche 13 ist in einem Arm 14 befestigt, der von einem dritten Halter 15 auf der optischen Bank 1 gehalten ist. Der dritte Halter 15 weist weiterhin einen Ausleger 16 auf, der mit dem Gehäuse und dem Mikroskoptubus 18 eines Mikroskopes 17 verbunden ist, das in üblicher Weise über einen Radantrieb 19 für eine Feinjustierung und einen Radantrieb 20 für eine Grobjustierung verfügt.

In der Zeichnung sind in das Mikroskop 17 die Lage diverser Linsen und des Strahlengangs eingezeichnet. Das Mikroskop 17 verfügt über ein Objektiv 21, dessen Eintrittslinse je nach dessen Brennweite in einem Abstand von 1 bis 100 mm, insbesondere 1 bis 2 mm, von der Endfläche 13 der Multimodefaser 10 angeordnet ist. Das Objektiv 21 hat beispielsweise eine numerische Apertur von 0,45 und erzeugt in der Nähe des Okulars 22 des Mikroskopes 17 ein Zwischenbild 23, das gegenüber der vom Objektiv 21 abgebildeten Endfläche 13 zwanzigfach vergrössert ist. Da der Kern der Multimodefaser 10 gleichmässig ausgeleuchtet ist, während die Stirnfläche des Mantels der Multimodefaser 10 dunkel bleibt, hat das Zwischenbild 23 in radialer Richtung gesehen eine rechteckförmige Lichtintensitätsverteilung entsprechend derjenigen unmittelbar auf der Endfläche 13 der Multimodefaser 10.

Das Zwischenbild 23 und damit die Endfläche 13 der Multimodefaser 10 wird mit Hilfe des Okulars 22 des Mikroskopes 17 als bestrahlte Fläche 7 auf die Arbeitsfläche 6 abgebildet. Wenn das Okular 22 des Mikroskopes eine Vergrösserung von 12 hat, gestattet es das Mikroskop 17 somit, eine Projektion der ausgangsseitigen Endfläche 13 der Multimodefaser 10 auf die Arbeitsfläche 6 mit einer Gesamtvergrösserung von 240 vorzunehmen. Bei einem Kerndurchmesser von 200 Mikrometern hat die bestrahlte Fläche 7 einen Durchmesser von 4,8 Zentimetern. Der Lichteinfall auf die bestrahlte Fläche 7 ist im wesentlichen lotrecht. Die optische Dosis ist sehr gleichmässig verteilt und fällt am Rand der bestrahlten Fläche 7 sprunghaft ab. Auf diese Weise kann die optische Dosis sehr präzise gleichmässig und reproduzierbar lokalisiert werden. Das Lot auf die bestrahlte Fläche 7 fluchtet dabei mit der Längsachse des Mikroskopes 17 und mit dem Lot auf die Endfläche 13 am im Arm 14 eingespannten Ende der Multimodefaser 10.

Um eine genaue Ausrichtung der Längsachse der Multimodefaser 10 im Bereich der Endfläche 13 und eine rechtwinklig zur Längsachse des Objektives 21 ausgerichtete Endfläche 13 zu erhalten, verfügt der Arm 14 über eine geeignete in der Zeichnung nicht dargestellte Halterung.

Der Fachmann erkennt aus der obigen Beschreibung, dass der Grundgedanke der Vorrichtung darin besteht, eine Abbildung der homogen bestrahlen Endfläche 13 der Multimodefaser 10, insbesondere einer Stufenindexfaser, auf der Arbeitsfläche 6 zu erzeugen, die die Hautoberfläche eines Patienten bzw. eines Tieres oder eine Fläche in einer Petrischale ist.

## Patentansprüche

1. Vorrichtung zum Homogenisieren der inhomogenen Lichtverteilung eines Laserstrahllichtbündels für eine scharfrandige Bestrahlung einer Fläche, mit einer vom Laserstrahllichtbündel beaufschlagten, eine Multimodefaser aufweisenden optischen Anordnung, wobei die optische Anordnung eine einige Meter lange Multimodefaser (10) aufweist, deren eingangsseitige Stirnfläche (11) von dem mit Hilfe einer Fokussieroptik (4) fokussierten Laserstrahllichtbündels (5) beaufschlagt ist und deren ausgangsseitige Endfläche (13) mit Hilfe einer vergrössernden Projektionsoptik (17) auf die zu bestrahlende Fläche (6, 7) abbildbar ist, dadurch gekennzeichnet, dass die vergrössernde Projektionsoptik ein Mikroskop (17) ist, dessen Objektiv (21) im geringen Abstand vor der Endfläche (13) der Multimodefaser (10) mit miteinander fluchtenden Längsachsen angeordnet ist.

2. Vorrichtung nach Anspruch 1, dadurch gekennzeichnet, dass das Objektiv (21) des Mikroskops (17) für eine Multimodefaser (10) mit einem Kerndurchmesser von 200 Mikrometern eine numerische Apertur von 0,45 aufweist.

3. Vorrichtung nach Anspruch 1 oder 2, dadurch gekennzeichnet, dass durch das Mikroskopobjektiv (21) ein zwanzigfach vergrössertes Zwischenbild (23) der Endfläche (13) der Multimodefaser (10) erzeugbar ist, das durch das Okular (22) des Mikroskopes (17) mit einer zwölffachen Vergrösserung auf die zu bestrahlende Fläche (6, 7) abbildbar ist.

4. Vorrichtung nach einem der vorstehenden Ansprüche, dadurch gekennzeichnet, dass die Fokussieroptik ein Mikroskopobjektiv (4) mit einer numerischen Apertur von 0,45 und einem Vergrösserungsfaktor von 20 ist.

5. Vorrichtung nach einem der Ansprüche 1 bis 4, dadurch gekennzeichnet, dass das Mikroskop (17) Einrichtungen (19, 20) zum Grobjustieren und Feinjustieren aufweist.

6. Vorrichtung nach einem der vorstehenden Ansprüche, dadurch gekennzeichnet, dass die Projektionsoptik (17) zusammen mit dem die ausgangsseitige Endfläche (13) aufweisenden Abschnitt der Multimodefaser (10) an einer Halteeinrichtung (14, 15, 16) befestigt ist, die in axialer Richtung gegenüber sowie parallel zu der beleuchteten Fläche (6, 7) verschiebbar ist.

7. Vorrichtung nach einem der vorstehenden Ansprüche, dadurch gekennzeichnet, dass die Multimodefaser (10) einen zu einer Spirale (12) aufgewickelten Abschnitt aufweist.

8. Vorrichtung nach einem der vorstehenden Ansprüche, dadurch gekennzeichnet, dass ein zwischen der Stirnfläche (11) und der Endfläche (13) liegender Abschnitt der Multimodefaser (10) an eine Vibrationseinrichtung mechanisch angekoppelt ist.

9. Vorrichtung nach Anspruch 8, dadurch gekennzeichnet, dass die Vibrationseinrichtung eine Biegeschwingungen erzeugende, hin und her rotierend schwingende Welle aufweist, deren Längsachse sich parallel zu einem kurzen Abschnitt der Multimodefaser (10) erstreckt und deren Ende mit dem Mantel der Multimodefaser (10) verbunden ist.

## Claims

1. Apparatus for homogenising the non-homogeneous light distribution of a laser beam for the sharp-edged irradiation of an area, having an optical arrangement struck by the laser beam that contains a multimode fibre, wherein the optical arrangement has a multimode fibre (10) several metres in length, the inlet end face (11) of which is struck by a laser beam (5) focussed by means of focussing optics (4) and the outlet end face (13) of which can be imaged onto the area (6, 7) to be irradiated using magnifying projection optics (17), wherein the magnifying projection optics are a microscope (17), of which the objective (21) is arranged a short distance in front of the end face (13) of the multimode fibre (10) with longitudinal axes aligned with one another.

2. Apparatus according to claim 1, wherein the objective (21) of the microscope (17) for a multimode fibre (10) having a core diameter of 200 micrometres has a numerical aperture of 0.45.

3. Apparatus according to claim 1 or 2, wherein it is possible to produce by means of the microscope objective (21) an intermediate image (23) of the end face (13) of the multimode fibre (10) magnified by a factor of twenty, which image can be projected by the eyepiece (22) of the microscope (17) with a magnification of 12 onto the area (6,7) to be irradiated.

4. Apparatus according to any one of the preceding claims, wherein the focussing optics are a microscope objective (4) having a numerical aperture of 0.45 and a magnification factor of 20.

5. Apparatus according to any one of claims 1 to 4, wherein the microscope (17) has means (19,20) for coarse adjustment and fine adjustment.

6. Apparatus according to any one of the preceding claims, wherein the projection optics (17), together with the portion of the multimode fibre (10) containing the outlet end face (13), are secured to a holding means (14, 15, 16) that is axially displaceable with respect to and parallel to the illuminated area (6,7).

7. Apparatus according to any one of the preceding claims, wherein a portion of the multimode fibre (10) is coiled in the form of a spiral (12).

8. Apparatus according to any one of the preceding claims, wherein a portion of the multimode fibre (10) lying between the front face (11) and the end face (13) is mechanically coupled to a vibration means.

9. Apparatus according to claim 8, wherein the vibration means has a shaft executing a reciprocal rotary vibration producing flexional vibrations, the longitudinal axis of which runs parallel to a short portion of the multimode fibre (10) and the end of which is joined to the cladding of the multimode fibre (10).

## Revendications

1. Appareil pour homogénéiser la distribution de lumière non-homogène d'un faisceau de lumière laser en vue d'une irradiation à bords nets d'une surface, comprenant un dispositif optique auquel est appliqué le faisceau de lumière laser et qui présente une fibre multimode, appareil dans lequel le dispositif optique comporte une fibre multimode (10) d'une longueur de quelques mètres, dont la face de tête (11) côté entrée est exposée au faisceau de lumière laser (5) focalisé à l'aide d'une optique de focalisation (4), et dont la face terminale (13) côté sortie peut être reproduite à l'aide d'une optique de projection (17) grossissante sur la surface (6, 7) à irradier, caractérisé en ce que l'optique de projection grossissante est un microscope (17) dont l'objectif (21) est placé à une faible distance devant la face terminale (13) de la fibre multimode (10), de manière que leurs axes longitudinaux soit mutuellement alignés.

2. Appareil selon la revendication 1, caractérisé en ce que l'objectif (21) du microscope (17) présente une ouverture numérique de 0,45 pour une fibre multimode (10) ayant un diamètre de coeur de 200 micromètres.

3. Appareil selon la revendication 1 ou 2, caractérisé en ce que l'objectif (21) du microscope est capable de produire une image intermédiaire (23) agrandie vingt fois de la face terminale (13) de la fibre multimode (10), image intermédiaire qui peut être reproduite par l'oculaire (22) du microscope (17) avec un grandissement de douze fois sur la surface (6, 7) à irradier.

4. Appareil selon une des revendications précédentes, caractérisé en ce que l'optique de focalisation est un objectif de microscope (4) ayant une ouverture numérique de 0,45 et un grossissement de 20.

5. Appareil selon une des revendications 1 à 4, caractérisé en ce que le microscope (17) comporte des dispositifs (19, 20) pour l'ajustement grossier et l'ajustement fin.

6. Appareil selon une des revendications précédentes, caractérisé en ce que l'optique de projection (17) est fixée, ensemble avec la partie de la fibre multimode (10) présentant la face terminale (13) côté sortie, sur un dispositif de maintien (14, 15, 16) qui est déplaçable axialement en translation par rapport ainsi que parallèlement à la surface (6, 7) à illuminer.

7. Appareil selon une des revendications précédentes, caractérisé en ce que la fibre multimode (10) comporte une partie enroulée en une spirale (12).

8. Appareil selon une des revendications précédentes, caractérisé en ce qu'une partie de la fibre multimode (10) située entre la face de tête (11) et la face terminale (13) est accouplée mécaniquement à un dispositif de vibration.

9. Appareil selon la revendication 8, caractérisé en ce que le dispositif de vibration comporte un arbre effectuant un mouvement rotatif d'oscillation en va-et-vient et générant des oscillations de flexion, arbre dont l'axe longitudinal s'étend parallèlement à un court tronçon de la fibre multimode (10) et dont l'extrémité est reliée à la gaine de la fibre multimode (10).
